# FASCICULE DE BREVET EUROPEEN

(11) **EP 2 446 859 B1**
(45) Date de publication et mention de la délivrance du brevet: **30.04.2014**
(21) Numéro de dépôt: 11306376.2
(22) Date de dépôt: 25.10.2011
(51) Int. Cl.: A61F 2/40, A61F 2/30

(54) **Implant glénoïdien.**
Glenoidales Implantat
Glenoid implant.

(30) Priorité: 28.10.2010 FR 1058907
(43) Date de publication de la demande: 02.05.2012
(73) Titulaire: Les Laboratoires Osteal Medical, 95957 Roissy Aeroport CDG (FR)
(72) Inventeur: Billuart, Fabien, 75014 Paris (FR)
(74) Mandataire: CAPRI

(56) Documents cités:
- EP-A1- 1 776 935
- EP-A1- 2 263 613
- EP-A2- 0 339 530
- EP-A2- 0 664 108

## Description

La présente invention concerne un implant glénoïdien qui est un élément de prothèse destiné à être implanté dans l'omoplate (scapula) et à coopérer par contact (roulement et/ou frottement) avec un autre élément de prothèse, à savoir une tête humérale montée sur une tige fixée dans l'humérus. Ces deux éléments de prothèse constituent une prothèse totale anatomique d'épaule destinée d'un côté à reproduire la glène de l'omoplate et de l'autre côté la tête de l'humérus qui se déplace avec des composantes complexes sur la surface de la glène.

On connaît déjà depuis longtemps certains problèmes liés à la mise en oeuvre de telles prothèses totales anatomiques d'épaule, et notamment au niveau de l'implant glénoïdien. En effet, l'implant glénoïdien peut être sujet à des descellements (mesurés radiologiquement à différents endroits de l'implant glénoïdien) avec apparition de liserés (jeu entre l'implant et l'os). On peut estimer le taux de descellement à environ 30 %.

A l'origine des prothèses d'épaule, il était d'usage de réaliser les surfaces de contact de l'implant glénoïdien et de la tête humérale avec des rayons de courbure correspondants, mais cela a entraîné un liseré précoce dans bon nombre de cas.

Il est actuellement recommandé d'utiliser des prothèses d'épaule dont les surfaces de contact de l'implant glénoïdien et de la tête humérale présentent des rayons de courbure non correspondants ou non congruents (mismatch), afin de reproduire la translation naturelle de la tête humérale sur la glène. Certains auteurs ont décrit une amélioration du taux de descellement avec une non correspondance (mismatch) des rayons de courbure de la tête humérale et de l'implant glénoïdien de plus de 5 mm. On a également remarqué que le liseré est d'autant moins important que le mismatch est adapté (un mismatch idéal étant aux alentours de 7 mm). L'utilisation d'un mismatch voisin de 7 mm n'est pas la solution idéale. En effet malgré la différence de rayon de courbure entre les implants il existe un phénomène d'usure du polyéthylène qui perdure. Ces usures proviennent notamment du fait qu'il se produit un effet dit de « rocking chair » qui combine des déplacements à la fois en rotation et en translation de la prothèse humérale dans la glène, par exemple pendant l'élévation et l'abaissement du bras. Cet effet de« rocking chair » est à l'origine de la plupart des descellements de l'implant glénoïdien.

La présente invention a pour but de surmonter ou de réduire les inconvénients précités de l'art antérieur en définissant un implant glénoïdien pour lequel le risque de descellement et/ou de production de débris d'usure est considérablement réduit. La présente invention s'applique plus particulièrement à un implant glénoïdien dont la surface de contact est congruente et de préférence réalisée en polyéthylène, mais il peut également s'appliquer à d'autres types de matériaux constitutifs de la surface de l'implant. Un autre but de la présente invention est de réduire la translation humérale et de contribuer au recentrage de la tête de l'humérus au centre de la glène. Encore un autre but est de réduire les phénomènes d'usure de matière causés par les translations de la tête humérale.

Dans l'art antérieur, on connaît déjà le document EP-2 263 613 qui décrit un implant glénoïdien selon le préambule de la revendication 1, la surface de tête définissant une périphérie externe pourvue d'un rebord.

Le but de la présente invention est d'augmenter la résilience ou souplesse de ce rebord et d'optimiser son positionnement sur la périphérie de la surface de tête.

Pour atteindre ces différents buts, la présente invention propose un implant glénoïdien ayant les caractéristiques de la revendication 1. Chaque segment de rebord peut être assimilé ou comparé à une petite ailette qui s'étend sur le pourtour de la surface de tête de l'implant glénoïdien. Les ailettes sont indépendantes les unes des autres, de sorte qu'une ailette peut être déformée sans affecter les autres. La segmentation du rebord augmente sa déformabilité locale. Ainsi, la tête de l'humérus qui se déplace par rotation et/ou translation sur la surface de tête de l'implant glénoïdien va venir en contact de ce rebord lorsqu'elle s'approche de la périphérie externe de la surface de tête. Plutôt que d'user la périphérie externe de la surface de tête, la tête de l'humérus va venir en butée contre le rebord saillant, qui remplace le bourrelet glénoïdien ou labrum naturel. Le contact de la tête de l'humérus avec ce rebord élastiquement déformable contribue au recentrage de la tête sur la surface de tête de l'implant glénoïdien, particulièrement lors de translation de la tête vers la périphérie de la surface de tête.

Avantageusement, le rebord fait saillie vers l'extérieur par rapport à la périphérie externe de la surface de tête. Ainsi, le rebord, qui fait office de labrum, s'étend à la fois vers le haut et vers l'extérieur de la périphérie externe de la surface de tête. On peut également dire qu'il prolonge de manière discontinue la surface de tête sur cette périphérie externe de manière à réaliser une butée pour la tête de l'humérus.

Selon un autre aspect de l'invention, le rebord s'étend sur une partie de la périphérie externe de la surface de tête. En variante, le rebord peut également s'étendre sur la totalité de la périphérie externe de la surface de tête. De préférence, la surface de tête définit, lorsqu'en place sur une omoplate, une zone supérieure et une zone inférieure, le rebord s'étendant dans la zone supérieure.

Selon une caractéristique pratique, le rebord est raccordé à la périphérie externe de la surface de tête par une partie amincie pour conférer de l'élasticité au rebord. Avantageusement, la surface de tête est formée par une coupelle définissant un bord périphérique, une gorge étant formée dans le bord périphérique pour réaliser la partie amincie. Dans ce cas, la gorge s'étend sous la surface de tête sur au moins une partie de la périphérie externe. De préférence, les fentes s'étendent jusqu'au niveau de la gorge. Ainsi, la partie amincie formée par la gorge a pour but d'améliorer les caractéristiques de déformation élastique du rebord pour amortir le contact de la tête de l'humérus avec le rebord.

Selon une technique de fabrication, l'implant glénoïdien peut être réalisé de manière monobloc en polyéthylène, et avantageusement en polyéthylène à poids moléculaire ultra haut (UHMWPE).

L'esprit de l'invention réside dans le fait de recréer de manière artificielle le bourrelet glénoïdien ou labrum naturellement présent sur la glène de l'omoplate. Pour lui conférer une certaine élasticité, le rebord est relié de manière appropriée au restant de l'implant glénoïdien.

L'invention sera maintenant plus amplement décrite en référence aux dessins joints donnant à titre d'exemple non limitatif un mode de réalisation de l'invention.

Sur les figures :
La figure 1 est une vue en perspective d'un implant glénoïdien selon un mode de réalisation de la présente invention,
La figure 2 est une vue de dessus de l'implant de la figure 1,
La figure 3 est une vue latérale de l'implant de la figure 1,
La figure 4 est une vue de dessous de l'implant de la figure 1,
La figure 5 est une vue en coupe de l'implant selon la ligne AA de la figure 1, et
La figure 6 est une vue en coupe de l'implant selon la ligne BB de la figure 1.

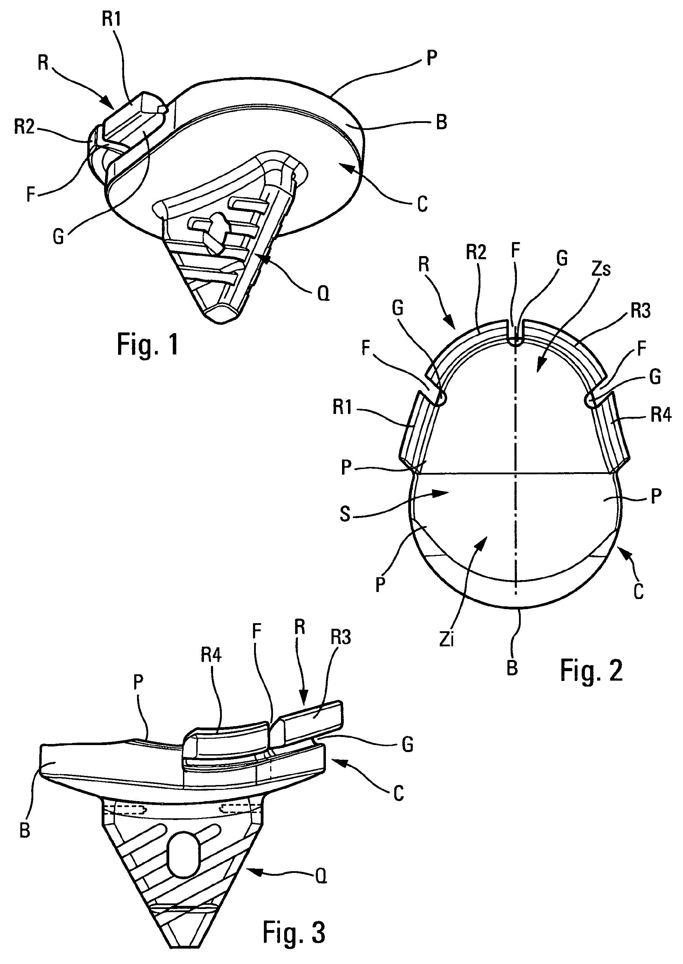

L'implant glénoïdien représenté sur les figures 1 à 6 est de préférence réalisé de manière monobloc en matière plastique, comme par exemple du polyéthylène, et avantageusement du polyéthylène à poids moléculaire ultra haut (UHMWPE).

Le procédé de fabrication utilise de préférence l'usinage, qui est préféré dans la plupart des cas à l'injection moulage. En variante non représentée, l'implant glénoïdien peut également être réalisé à partir de plusieurs matériaux différents par des techniques de bi-injection ou de surmoulage. Ceci n'est pas critique pour la présente invention.

L'implant glénoïdien des figures comprend essentiellement deux parties reliées ensemble de manière monobloc, à savoir un talon d'ancrage Q et une coupelle C.

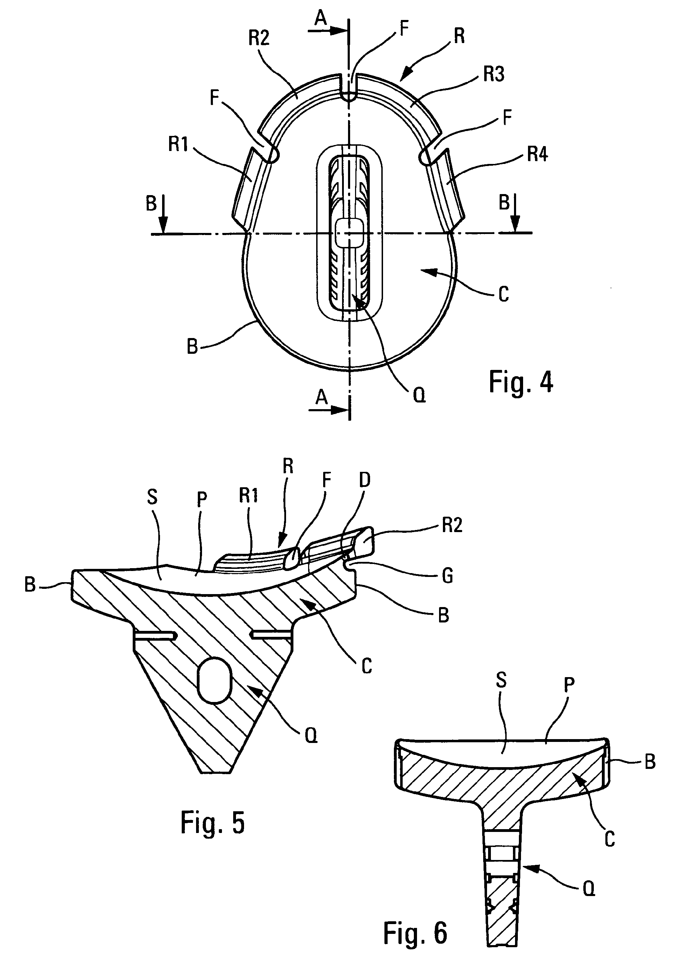

Le talon d'ancrage Q a pour fonction, comme son nom l'indique, d'assurer l'ancrage de l'implant glénoïdien dans l'os de l'omoplate. Le talon Q est scellé dans un évidement pratiqué dans l'os de l'omoplate à l'aide d'un ciment (pmma). Pour favoriser l'accrochage sur le talon Q, celui-ci peut présenter des profils appropriés. Comme on peut le voir à partir des figures, le talon d'ancrage Q présente une configuration mince, étroite ou aplatie dans le plan transversal (figure 6). En revanche, dans le plan longitudinal (figures 3 et 5), le talon Q présente une configuration générale triangulaire avec un sommet pointant vers le bas et un côté opposé supérieur raccordé à la coupelle C. L'orientation classique du talon d'ancrage Q permet de déterminer l'orientation du plan transversal et du plan perpendiculaire longitudinal.

La coupelle C comprend une surface inférieure d'appui qui entoure le talon d'ancrage Q. Cette surface d'appui a pour fonction de reposer sur l'os de l'omoplate qui a été préalablement fraisé avec un rayon de courbure unique déterminé, qui peut par exemple être de 42,7 mm. C'est pourquoi la surface d'appui présente également un rayon de courbure unique qui est sensiblement ou parfaitement égal à celui du fraisage de l'os de l'omoplate. La coupelle C définit également un bord périphérique B de configuration sensiblement cylindrique mais non circulaire, comme on peut le voir à partir des figures 1, 2 et 4. En effet, on peut remarquer que le bord B présente une configuration générale sensiblement ovoïdale ou en forme de poire avec une partie inférieure plus étendue que la partie supérieure.

La coupelle C comprend également une surface supérieure ou surface de tête S qui présente une forme générale concave, comme on peut clairement le voir sur les figures 5 et 6. La surface de tête S est celle contre laquelle la tête de l'humérus vient en contact de rotation et/ou de frottement. Cette surface S peut correspondre à un ou plusieurs rayon(s) de courbure distinct(s) dans le sens transversal et longitudinal. De préférence, la surface de tête est dite non congruente (mismatch) de sorte qu'elle présente globalement un rayon de courbure supérieur à celui de la tête de l'humérus, par exemple de l'ordre de 5 à 9 mm. Il s'agit là d'une caractéristique désormais classique pour un implant glénoïdien. La surface de tête S présente une périphérie extérieure P de forme générale ovoïdale ou en forme de poire, comme on peut le voir sur la figure 2. Dans sa partie inférieure, la surface S se raccorde à une plage sensiblement plane en forme de croissant de lune. La périphérie externe P se raccorde au bord périphérique B, hormis au niveau de la partie inférieure de la surface de tête au niveau de la plage plane.

Selon l'invention, la périphérie externe P est pourvue d'un rebord R qui fait saillie vers l'extérieur et vers le haut par rapport à la surface de tête S. Ceci est clairement visible sur les figures 3 et 5. Ce rebord R prolonge ainsi de manière discontinue la surface de tête S au niveau de sa périphérie externe P. Le rebord R empêche un raccordement direct de la périphérie externe P avec le bord périphérique B. On peut ainsi dire que le rebord R s'étend au niveau de la jonction de la périphérie externe P et du bord périphérique B. Comme on peut le voir sur la figure 5, le rebord R crée un bossage saillant par rapport à la courbure continue et lisse de la surface de tête S. De cette manière, le rebord R va faire fonction de butée périphérique pour la tête de l'humérus qui se déplace en rotation et translation sur la surface de tête S. Ce rebord R remplit ainsi la fonction naturelle du bourrelet glénoïdien ou labrum.

Le rebord R peut être continu sur toute ou partie de la périphérie externe P de la surface S. Avantageusement, le rebord R ne s'étend que sur une partie de la périphérie, comme on peut le voir sur les figures. Plus précisément, on peut diviser la surface de tête S en une zone supérieure Zs et une zone inférieure Zi, qui correspond à l'orientation de l'implant glénoïdien sur une omoplate. Ainsi défini, on peut remarquer que le rebord R ne s'étend que sur la zone supérieure Zs. La zone inférieure Zi est ici dépourvue de rebord R. Toutefois, on peut également imaginer que le rebord R s'étend également sur tout ou partie de la zone inférieure Zi. D'autre part, on peut également constater que le rebord R ne s'étend pas de manière continue, mais forme plusieurs segments de rebord R1, R2, R3 et R4 qui sont séparés par des fentes radiales F. En se référant aux figures 1 et 2, on peut remarquer que le rebord R comprend deux segments latéraux R1 et R4 et deux segments supérieurs R2 et R3. Il s'agit là d'une configuration avantageuse, mais cependant non limitative. On peut en effet imaginer de séparer le rebord R en un nombre différent de segments séparés par des fentes F disposés autrement. La segmentation du rebord R a pour fonction de lui conférer une élasticité et une plus grande indépendance de déformation. En effet, lorsque la tête de l'humérus vient en butée contre le segment R2, les autres segments ne sont que très peu affectés, du fait que le segment R2 est séparé des segments adjacents par des fentes F. On peut à cet égard remarquer que les fentes F s'étendent jusqu'au niveau de la périphérie extérieure P de la surface de tête S, et l'entame même partiellement.

Selon une autre caractéristique intéressante de l'invention, le bord périphérique B est pourvu d'une gorge périphérique G qui s'étend sur toute ou partie de la périphérie du bord B. Dans le cas présent, la gorge G ne s'étend que dans la zone supérieure Zs, là où se trouve le rebord R. La gorge G est formée juste en dessous de la périphérie externe P de la surface de tête S, comme on peut clairement le voir sur la figure 5. Ainsi, une partie amincie D est formée au niveau où la gorge G se rapproche de la surface de tête S. La partie amincie D a également pour fonction d'augmenter les caractéristiques de déformabilité élastiques du rebord R, et plus particulièrement des segments R1 à R4. On peut même constater sur la figure 2 que les fentes F s'étendent jusque dans la gorge G. De cette manière, les segments R1 à R4 sont totalement indépendants et peuvent se déformer individuellement sans affecter les segments adjacents.

Le rebord R est de préférence réalisé de manière monobloc avec le restant de l'implant glénoïdien en étant relié à celui-ci par la partie amincie D. L'ajout du rebord R, par rapport à un implant glénoïdien classique, n'entraîne aucune difficulté de fabrication, ni de mise en place.

Grâce à cet implant glénoïdien, le recentrage de la tête de l'humérus est amélioré par contact avec le rebord R, ce qui conduit une baisse considérable des risques de descellement de l'implant glénoïdien.

## Revendications

1. Implant glénoïdien destiné à être implanté dans l'omoplate et à coopérer par roulement et/ou frottement avec une tête humérale, l'implant glénoïdien comprenant une surface de tête (St) de forme générale concave sur laquelle la tête humérale vient en contact de roulement et/ou de frottement, la surface de tête (S) définissant une périphérie externe (P) pourvue d'un rebord (R) qui fait saillie vers le haut par rapport à la surface de tête (S), **caractérisé en ce que** le rebord (R) est discontinu, de manière à former des segments de rebord (R1, R2, R3, R4) séparés par des fentes (F).

2. Implant glénoïdien selon la revendication 1, dans lequel le rebord (R) est formé de quatre segments de rebords (R1, R2, R3, R4) séparés par trois fentes (F), à savoir deux segments latéraux (R1, R4) et deux segments supérieurs (R2, R3).

3. Implant glénoïdien selon la revendication 1 ou 2, dans lequel le rebord (R) fait saillie vers l'extérieur par rapport à la périphérie externe (P) de la surface de tête (S).

4. Implant glénoïdien selon la revendication 1, 2 ou 3, dans lequel le rebord (R) s'étend sur une partie de la périphérie externe (P) de la surface de tête (S).

5. Implant glénoïdien selon la revendication 4, dans lequel la surface de tête (S) définit, lorsqu'en place sur une omoplate, une zone supérieure (Zs) et une zone inférieure (Zi), le rebord (R) s'étendant dans la zone supérieure (Zs).

6. Implant glénoïdien selon l'une quelconque des revendications précédentes, dans lequel le rebord (R) est raccordé à la périphérie externe (P) de la surface de tête (S) par une partie amincie (D) pour conférer de l'élasticité au rebord (R).

7. Implant glénoïdien selon la revendication 6, dans lequel la surface de tête (S) est formée par une coupelle (C) définissant un bord périphérique (B), une gorge (G) étant formée dans le bord périphérique (B) pour réaliser la partie amincie (D).

8. Implant glénoïdien selon la revendication 7, dans lequel la gorge (G) s'étend sous la surface de tête (S) sur au moins une partie de la périphérie externe (P).

9. Implant glénoïdien selon la revendication 7 ou 8, dans lequel les fentes (F) s'étendent jusqu'au niveau de la gorge (G).

10. Implant glénoïdien selon l'une quelconque des revendications précédentes, réalisé de manière monobloc en polyéthylène, avantageusement en polyéthylène à poids moléculaire ultra haut (UHMWPE).

## Patentansprüche

1. Gelenkimplantat zur Implantation in das Schulterblatt und zur Zusammenarbeit durch Rollen und/oder Reiben mit einem Oberarmkopf, wobei das Gelenkimplantat eine Kopfoberfläche (St) umfasst, dessen allgemeine Form konkav ist, auf der der Oberarmkopf in Kontakt durch Rollen und/oder Reiben ist, wobei die Oberfläche des Kopfs (S) eine externe Peripherie (P) definiert, die über einen Rand (R) verfügt, der nach oben über die Oberfläche des Kopfs (S) herausragt, **dadurch gekennzeichnet, dass** der Rand (R) unterbrochen ist, so dass Segmente des Rands (R1, R2, R3, R4) ausgebildet werden, die durch Kerben (F) getrennt sind.

2. Gelenkimplantat gemäß Anspruch 1, bei dem der Rand (R) aus vier Segmenten des Rands (R1, R2, R3, R4) besteht, die durch drei Kerben (F) voneinander getrennt sind, d. h. zwei seitliche Segmente (R1, R4) und zwei obere Segmente (R2, R3).

3. Gelenkimplantat gemäß Anspruch 1 oder 2, bei dem der Rand (R) nach Außen übersteht, im Verhältnis zur externen Peripherie (P) der Oberfläche des Kopfs (S).

4. Gelenkimplantat gemäß Anspruch 1, 2 oder 3, bei dem der Rand (R) sich auf einen Teil der externen Peripherie (P) der Oberfläche des Kopfs erstreckt.

5. Gelenkimplantat gemäß Anspruch 4, bei dem die Oberfläche des Kopfs (S), wenn er sich an seinem Platz auf dem Schulterblatt befindet, einen oberen Bereich (Zs) und einen unteren Bereich (Zi) ausbildet, wobei sich der Rand (R) in den oberen Bereich (Zs) ausdehnt.

6. Gelenkimplantat gemäß einem der vorangegangenen Ansprüche, bei dem der Rand (R) an die externe Peripherie (P) der Oberfläche des Kopfs (S) mittels eines sich verjüngenden Teils (D) angeschlossen wird, um dem Rand (R) Elastizität zu verleihen.

7. Gelenkimplantat gemäß Anspruch 6, bei dem die Oberfläche des Kopfs (S) durch eine Manschette (C) gebildet wird, die einen umfänglichen Rand definiert(B), und durch eine Rille (G), die in dem umfänglichen Rand (B) ausgebildet ist, um das sich verjüngende Teil (D) auszuführen.

8. Gelenkimplantat gemäß Anspruch 7, bei dem die Rille (G) unter der Oberfläche des Kopfs (S) sich auf mindestens einen Teil der externen Peripherie (P) erstreckt.

9. Gelenkimplantat gemäß Anspruch 7 oder 8, bei dem die Kerben (F) sich bis zur Höhe der Rille (G) erstrecken.

10. Gelenkimplantat gemäß einem der vorangegangenen Ansprüche, ausgeführt als Monoblock aus Polyethylen, bevorzugt aus ultrahochmolekularem Polyethylen (UHMWPE).

## Claims

1. A glenoid implant intended to be implanted in the scapula and to cooperate by rolling and/or rubbing with a humeral head, the glenoid implant comprising a head surface (St) with a general concave shape on which the humeral head comes into rolling and/or rubbing contact, the head surface (S) defining an external periphery (P) provided with an edge (R) which protrudes upwards relatively to the head surface (S), **characterized in that** the edge is discontinuous, so as to form edge segments (R1, R2, R3, R4) separated by slits (F).

2. The glenoid implant according to claim 1, wherein the edge (R) is formed with four edge segments (R1, R2, R3, R4) separated by three slits (F), i.e. two lateral segments (R1, R4) and two upper segments (R2, R3).

3. The glenoid implant according to claim 1 or 2, wherein the edge (R) protrudes outwards relatively to the external periphery (P) of the head surface (S).

4. The glenoid implant according to claim 1, 2 or 3, wherein the edge (R) extends over a portion of the external periphery (P) of the head surface (S).

5. The glenoid implant according to claim 4, wherein the head surface (S) defines, when in place on the scapula, an upper area (Zs) and a lower area (Zi), the edge (R) extending into the upper area (Zs).

6. The glenoid implant according to any of the preceding claims, wherein the edge (R) is connected to the external periphery (P) of the head surface (S) through a thinned portion (D) for giving elasticity to the edge.

7. The glenoid implant according to claim 6, wherein the head surface (S) is formed by a cup (C) defining a peripheral edge (B), a groove (G) being formed in the peripheral edge (B) for making the thinned portion (D).

8. The glenoid implant according to claim 7, wherein the groove (G) extends under the head surface (S) over at least one portion of the external periphery (P).

9. The glenoid implant according to claim 7 or 8, wherein the slits (F) extend as far as the level of the groove (G).

10. The glenoid implant according to any of the preceding claims, made as a single block in polyethylene, advantageously in ultra high molecular weight polyethylene (UHMWPE).
